(19) Europäisches Patentamt / European Patent Office / Office européen des brevets

(11) **EP 3 936 187 A1**

(12) **EUROPEAN PATENT APPLICATION**

(43) Date of publication:
**12.01.2022  Bulletin 2022/02**

(51) Int Cl.:
**A61N 5/10** (2006.01)

(21) Application number: **20184936.1**

(22) Date of filing: **09.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **BONDAR, Maria Luiza**
**5656 AE Eindhoven (NL)**

• **WEESE, Rolf Jürgen**
**5656 AE Eindhoven (NL)**
• **VIK, Torbjoern**
**5656 AE Eindhoven (NL)**
• **BROSCH, Tom**
**5656 AE Eindhoven (NL)**
• **WIEGERT, Jens**
**5656 AE Eindhoven (NL)**
• **HEESE, Harald Sepp**
**5656 AE Eindhoven (NL)**

(74) Representative: **Philips Intellectual Property & Standards**
**High Tech Campus 52**
**5656 AG Eindhoven (NL)**

(54) **AN ARTIFICIAL INTELLIGENCE SYSTEM TO SUPPORT ADAPTIVE RADIOTHERAPY**

(57) A computing system and related method for supporting in particular adaptive radiation therapy. An input interface (IN) of the system receives an input image. A machine learning module (MLM) predicts, based on the input image, a predicted dose distribution associated with a first planning technique or a first treatment modality. A comparator (COMP) compares a planned dose distribution as per a current treatment plan with the predicted dose distribution, to obtain a comparison result. The result allows user to gauge whether an actual re-planning would yield a dosimetric benefit before committing time or computational resources.

**FIG. 1**

**Description**

FIELD OF THE INVENTION

The invention relates to system for supporting radiation therapy, to a computing system for training a machine learning module for use in supporting radiation therapy, to a method of supporting radiation therapy, to a computer program element, and to a computer readable medium.

BACKGROUND OF THE INVENTION

Cancer is a leading cause of death in industrialized nations around the world. Well over half of cancer patients receive treatment by external beam Radiotherapy ("RT"). High energy ionizing radiation is applied to the patient, either from outside the patient (external RT) or from within (brachytherapy). The goal is to destroy cancerous tissue whilst preserving non-cancerous, healthy tissue. In some types of external RT, X-radiation in the megavolt (MV) range is used. The total radiation dose is administered in portions (also known as "fractions") over time as per a treatment plan, such as once per day over the course of 2-4 weeks for example.

During the course of a fractionated radiotherapy treatment, differences between the intended and the delivered dose can occur due to e.g., anatomical changes. Dose differences exceeding clinically accepted thresholds can be minimized using an adaptive radiotherapy workflow consisting of 1) assessment of delivered dose and of dose differences, 2) assessment of need for re-planning based on dosimetric criteria and dose thresholds, and 3) re-planning. Generally, the dosimetric criteria are based on clinical planning objectives and goals, eg, mean parotid dose $\leq$ 26 *Gy,* and mean heart dose $\leq$ 10 *Gy.*

In current clinical practice, the delivered dose can be estimated by recalculating the planned dose using images ("control images") acquired during the course of treatment. A common imaging modality used to assess anatomical changes is in-room acquired CBCT (cone beam computed tomography).CT, MRI (magnetic resonance imaging), or others can be also used. For the cases in which the dose deviations are greater than clinically accepted, the clinical user can decide to acquire a new planning image (commonly a CT-scan) and re-plan the treatment. This approach is sometimes referred to as adaptive radiotherapy.

Current practice in adaptive radiotherapy workflow suffers from a limitation in that the user may take the decision to acquire a re-planning image, and may request a re-planning for a new treatment, without actually knowing whether there indeed is a benefit in re-planning at all. Indeed, for some patients, re-planning might not reduce the dose deviation below a desired threshold and might have a limited clinical benefit.

The second limitation is that the clinical user may not have the means to forecast whether re-planning will be required for a patient and on which treatment day. Therefore, delays in treating the patient with a new plan may occur due to resource constraints.

SUMMARY OF THE INVENTION

There may therefore be a need to address at least some of the above noted limitations or to otherwise improve RT.

The object of the present invention is solved by the subject matter of the independent claims where further embodiments are incorporated in the dependent claims. It should be noted that the following described aspect of the invention equally applies to the computing system for training a machine learning module for use in supporting radiation therapy, to the computer program element and to the computer readable medium.

According to a first aspect of the invention there is provided a computing system for supporting radiation therapy, comprising:

- an input interface for receiving an input image;
- a (trained) machine learning module to predict based on the input image, a predicted dose distribution associated with a first planning technique and/or first treatment modality; and
- a comparator configured to compare a planned dose distribution as per a current treatment plan with the predicted dose distribution, to obtain a comparison result.

The result allows user to gauge whether an actual re-planning would yield a dosimetric gain/benefit before committing time or computational resources. The system is of particular benefit for adaptive RT.

In embodiments, the system includes a graphics display generator configured to cause a display device to display the comparison result or data derivable therefrom.

In embodiments, the said comparison result is displayed in association with the input image.

In embodiments the said comparison result is displayed globally for the whole input image or locally per image element. The element may be an individual pixel/voxel or a user definable sub-set of the image. This allows focusing the comparison on a particular region of interest.

In embodiments, in response to the comparison result, or in response to a user request, a re-planning module is to compute a new treatment plan. The comparison result may be displayed, and the user may then issue through a user interface the request for the re-planning if the result so suggests, for example, if a dosimetric gain/benefit can be had from re-planning.

In embodiments the system may include a scheduler configured to schedule a new image session to acquire a new planning image, and/or a new re-planning session using the same (the first) or a new (re-)planning technique, and/or a new treatment session with a new treatment modality.

In embodiments, the machine learning module is one of a plurality of such module, with different ones of the said plurality of machine learning modules respectively associated with different planning techniques and/or different treatment modalities, the said modules held in one or more data memories.

In embodiments, the system comprise a user interface for the user to select a different machine learning module from the said plurality, and the system to produce a new comparison result, based on the selected machine learning module.

In embodiments, the machine learning module, or a further machine learning module, is configured to predict an image representing anatomical changes due to applicable radiation fractions.

In embodiments, the comparison result is used by a treatment outcome predictor to estimate a treatment outcome, such as side effects, tumor control.

In another aspect there is provided a computing system for training, based on training data, a machine learning module as per any one of the previous claims.

In another aspect there is provided a computer-implemented method for supporting radiation therapy, comprising:

- receiving an input image;
- with a machine learning module , predicting, based on the input image, a predicted dose distribution associated with a first planning technique; and
- comparing a planned dose distribution as per a current treatment plan with the predicted dose distribution, to obtain a comparison result.

In another aspect there is provided a computer-implemented method of training, based on training data, a machine learning module as per any one of previous claims.

In another aspect there is provided a computer program element, which, when being executed by at least one processing unit, is adapted to cause the processing unit to perform the method as per any one of the above mentioned embodiments.

In another aspect still, there is provided a computer readable medium having stored thereon the program element or having stored thereon the trained machine learning module.

The proposed system and method allow predicting, with a machine learning ("ML") model (such as neural network(s)) at planning stage, patient anatomy and/or its corresponding (optimal) re-planned dose distribution, for any later fraction i (i = 1, ... ,n).The system and method allow allocating, at the planning phase, departmental resources based on the difference between the predicted optimal re-planned dose distribution and a recalculated planned dose distribution of the anatomy for a given future fraction. The proposed system and method allow supporting decision making for treatment adaptation by predicting via the pre-trained machine learning model the optimal re-planned dose from a control image at a current fraction $i$. For example, the system and method may support a user in deciding whether to acquire a new planning image, such as in a CT-scan for example. The proposed system and method allow predicting dose distribution close to the achievable (optimal) re-planned dose and to use the predicted dose distribution to support decisions for adaptation. The proposed solution is a fast and resource-inexpensive alternative to actual full re-planning based on in-room acquired imaging modalities for example. The proposed ML based prediction is computationally cheaper than the actual performance of an automated re-planning algorithm. The proposed system method thus allow time savings and electrical energy consumption may be reduced because unnecessary re-planning can be avoided if it is found the dosimetric benefit to be had from the re-planning is marginal or in fact non-existent. In addition, the proposed ML system can be configured to predict the benefit of re-planning by using other planning techniques and/or other radiation treatment modalities than the one(s) for the current treatment. In sum, the proposed system and method are configured to use ML to predict achievable optimized dose distribution, without actually performing the computationally expensive re-planning.

Optionally, the system or method proposed herein allow forecasting a suitable moment in time for re-planning for a patient. The system and method allow the clinical user to schedule time slots for image acquisition and re-planning. In the proposed system and method, such forecasting can be facilitated by predicting possible anatomical changes during the course of the treatment and/or their impact on the dose distribution.

<u>Definitions</u>

*"user"* relates to a person, such as medical personnel, clinician, or other, operating the RT delivery equipment, overseeing the RT procedure, drawing up a treatment plan or re-planning. In other words, the user is in general not the patient.

In general, the *"machine learning module"* is a computerized arrangement that implements a machine learning ("ML") algorithm that is configured to perform a task. The machine learning algorithm is based on an ML model. In an ML algorithm, task performance improves measurably after having provided the arrangement with more training data. The performance may be measured by objective tests when feeding the system with test data. The performance may be defined in terms of a certain error rate to be achieved for the given test data. See for example, T. M. Mitchell, "Machine Learning", page 2, section 1.1, McGraw-Hill, 1997.

BRIEF DESCRIPTION OF THE DRAWINGS

Exemplary embodiments of the invention will now be described with reference to the following drawings, which, unless stated otherwise, are not to scale, wherein:

Fig. 1 is a schematic block diagram of a radiation treatment system;
Fig. 2 shows a schematic block diagram of a re-planning decision support system according to one embodiment;
Figs. 3-5 show schematic block diagrams of re-planning decision systems according to furthers embodiments;
Fig. 6 shows a schematic block diagram of a machine learning model;
Fig. 7 shows a computerized training system for training a machine learning model;
Fig. 8 shows a flow chart of a method of supporting radiation therapy; and
Fig. 9 shows a flow chart of a method for training a machine learning model for supporting radiation therapy.

DETAILED DESCRIPTION OF EMBODIMENTS

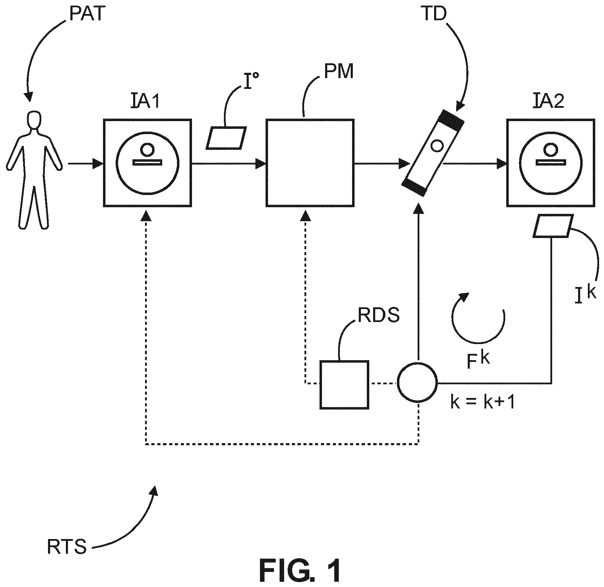

With reference to Fig. 1 there is shown a schematic block diagram of a computerized radiation therapy ("RT")-system RTS. External radiation therapy is mainly envisaged herein although the principles described herein may also be applied to internal radiation therapy. In external radiation therapy, high energy radiation is delivered to a patient to kill off cancerous tissue. The objective is to kill off as much of the cancerous tissue as possible whilst sparing other non-cancerous tissue. The cancer killing radiation is delivered by a treatment device TD such as a linear accelerator ("linac"). In linacs, high energy x-ray photons in the megavolt range are generated and the patient is exposed in a pre-mediated manner to the radiation delivered by the treatment device TD.

More particularly, in intensity-modulated radiation therapy ("IMRT") and volumetric-modulated arc therapy, types of X-ray based external RT envisaged herein in embodiments, the radiation is delivered at certain doses from a multitude of different spatial directions. Specifically, radiation treatment beams propagating along the said different spatial directions can be made to intersect preferably at the lesion to be treated, thus delivering a higher dose there, whilst leaving a lower dose at surrounding non-cancerous tissue, in particular organs at risk (OAR). The applicable doses to be delivered and the spatial directions relative to the lesion site through which the dosages are to be delivered are defined in a treatment plan. The treatment plan is computed by a planning module PM which may be implemented by a computing system.

Computing a treatment plan is in general a complex mathematical operation that may require time and electrical energy. Preferably, high performance computing processors such as those of multi-core design may be used to secure a reasonable return time. The time resources for patient and medical facilitates that be spent may also be considerable. In embodiments, GPUs are used to compute the treatment plan. Computing the treatment plan, the planning problem, may be formulated as an optimization. Specifically, one or more objective functions are formulated subject to one or more constraints. The objective function and the constraints define the manner in which the treatment dose is to be delivered given location, shape and extent of the cancerous lesion. More specifically, the objective function(s) and the constraint(s) describe how much dosage is to be delivered in which regions. A clinician prescribes for instance the average dose in a certain area or the maximum or minimum dose to be delivered and formulates these objectives and constraints. The objective function(s) is a 3D scalar field that assigned to each spatial point in treatment space, where the patient is located during treatment, a certain deliverable dose. The constraint(s) qualify the deliverable dose. An exemplary setup may be illustrated as objective to deliver a uniform dose of 60 Gy in 30 fractions, with the constraint to keep the dose below 25 Gy in more than 70% of the target volume of the structure and to spar dose exposure entirely, or at least restrict dose up to a threshold, at certain OARs, such as the parotid gland or the spinal cord for example.

The planning module PM then implements a planning algorithm. In particular inverse planning algorithms may be iterative and computes how much dosage is to be delivered from which spatial position over a certain period of time. In the iterations, the objective function is improved until an acceptable treatment plan is found. The total dose is delivered in fractionations. In other words, parts of the dose are delivered over a treatment course of days or weeks, in multiple treatment sessions. A treatment session for a fractionation $i$ is usually once per day. In each such treatment, a certain dose part of the total dose envisaged is delivered from the computed spatial positions. Such a dose part is referred to herein as the $i$-th fraction, or "fraction i".

In more detail, an RT treatment plan may include in particular a quantity known as a "fluence map". The fluence map $\varphi$ defines the intensity of the treatment beam across its cross section. More particularly, a treatment beam (also called an elementary beam), for a given radiation direction (as per the treatment head's position relative to the ROI) is comprised from single rays called "beamlets". Each elementary beam is determined by its angular position (or direction $\alpha$), its initial energy and its (2D) fluence map $\varphi_i$. The fluence map comprises elements called "bixels". The number of bixels in each fluence map is equal to the number of beamlets for the given elementary beam. Each bixel defines a "weight" (that is, a number) for a particular beamlet. This weight expresses the contribution of said beamlet to the dose delivered along said direction. In IMRT then, the total irradiated dose is composed by the superposition of several independent elementary beams, one static beam per each angular direction. Usually, between 5-14 (but this number is exemplary only) delivery directions are used in IMRT. The treatment plan may define treatment beam geometry and/or beam parameters.

The planning algorithm/technique facilitates computing an optimized fluence map of each lesion of patient. Based on the computed fluence map, a leaf sequencing tool computes the control program to control position and timing motions of the individual leaves of the MLF collimator. The computing of the fluence map is a computationally complex procedure that involves an optimization of the objective function. The optimization is constrained to account for the above- mentioned tradeoff between preservation of health issue and killing-off cancer tissue. The computation may be based on the segmented 3D CT image that defines the lesion. The computation of the fluence map amounts to solving a positivity-constrained optimization problem with known methods. See for instance Pflugfelder et al, "A comparison of three optimization algorithms for intensity modulated radiation therapy", Z. Med. Phys. (2008) Vol. 18, No. 2, pp. 111-119.

With continued reference to Fig. 1, the general work flow of radiation treatment may be described as follows: a patient PAT is examined by a clinician based on detailed medical imagery acquired by a medical image modality IA1, such as computed a tomography scanner CT, a magnetic resonance imager (MRI), or a nuclear imaging modality such as PET/SPECT. Based on this planning imagery $I^0$, the cancerous region is identified.

This initial imagery $I^0$ is preferably 3D so as to capture the 3D shape and extent of the cancer lesion in the patient. The lesion is identified either manually delineated in the imagery or the delineation is done automatically or semi-automatically by a segmentation algorithm. The identified tissue includes the volume to be treated where the cancerous tissue resides plus a certain safety margin. Volumes of Non-cancerous tissue, such as the above mentioned OAR may also be segmented. OARS are to be spared from radiation exposure, or deposited radiation is at least not to succeed certain maximum thresholds as formulated in the planning constraints.

Based on the initial planning imagery $I^0$, the clinician prescribes and formulates the overall dose objective subject to the constraints. This information is then fed into the planning module PM that uses the above described iterative procedure to compute the treatment plan. The treatment plan describes how much dosage is to be administered in multiple fractions and for each fraction describes the spatial directions through the target 3D volume along which the radiation is to be delivered by the treatment device TD.

The treatment plan may further includes, or is translatable into, a control program or machine parameters that control(s) the operation of the treatment device during delivery. For instance, in modulated radiation therapy the treatment device such as the linac includes a treatment head with a multi-collimator that can realize different beam shapes. The cross section of the beam may be divided into beamlets where different dosages can be realized in a spatially resolved manner. Control program of the treatment plan may prescribe different collimator settings that may vary as the treatment head moves to different spatial positions around the target volume. The collimator settings may prescribe how leaf elements of the multi-leaf collimator are to be moved to realize the different beam shapes. The patient PAT is then asked to lie on a treatment table for instance and the treatment device is operated to deliver dosage along the computed spatial directions as per treatment plan in a first treatment fraction $k$. This is then repeated over multiple days in different fractionations $F^k$ $k>1$. At some or each treatment day, control imagery $I^k$, $k \geq 1$ is acquired by preferably another imaging modality to monitor progress of the treatment. The control image IK is sometime called "image of day" although the frequency is not necessarily daily. In general, this monitoring imaging modality IA2, or "control imager" operates preferably at a lower dose than the initial imaging modality IA1 at which the initial planning image $I^0$ has been acquired. The control imagery allows the healthcare processional to monitor how the lesion responds to radiation. The control imaging modality IA2 may be a kV-in-room cone beam scanner CBT. These devices are frequently integrated into the treatment device. "In-room" referrers to the control imager IA2 being arranged in the treatment room together with the treatment device TD or that the control imager may is movable into the treatment room. Specifically, the control imager IA2 may be arranged as a permanent fixture in the treatment room, just like the linac or may be mobile, so can be moved into the room, on wheels or tracks or other, when required. Specifically, the in-room imaging apparatus IA1 may be integrated into the linac.

Radiation treatment in the described manner is not a static process. It is dynamic in the sense that the cancerous tissue, and also the organs at risk, may react to the delivered radiation and will change over the course of the fractions. For reasons not fully understood and individual to a patient, the cancerous tissue or the OARs may re-act differently than expected, and this may necessitate a re-planning of the treatment plan. This is particularly called for if the prescribed dosages at the organs at risk are to be exceeded, and/or if the prescribed dosage at the lesioned site cannot be delivered at the minimum required dosage for instance. In these instances, (schematically indicated by a circle in Fig. 1), it may be required to revisit the planning phase. Revisiting the planning phase may include for instance that another planning image $I^{0'}$ is acquired by the possibly high dose imaging modality IA1. A re-planning may be required which may include new or updated dosage objectives and/or constraints be formulated and to be fed again into the planning module to re-compute the plan.

It has been observed however, that re-computing a treatment plan may not always lead to a dosimetric benefit one had hoped for. In other words, at times one finds that despite having spent time and hospital resources in acquiring new planning imagery and re-planning the treatment plan, the new plan with the re-planned dose does not in fact markedly improve the intended dose distribution. For instance, it may still be the case that OAR receive higher dosage and/or that the target volume still does not receive sufficient dose. These situations are unfortunate as patient burden increases, hospital resources are strained, and electrical energy have been expended in an essentially futile exercise. In particular, the computation of the plan can draw considerable electrical energy because of the use of high performance computing equipment that in turn causes a considerable $CO_2$ footprint when scaled up across multiple oncology departments and patient number, the later set to rise given the ever aging population in many demographics around the world. In addition, a new planning image typically means a new CT scan for example. This means that a new time slot at the CT-scan needs to be booked, the patient needs a new appointment, etc.

It is therefore proposed herein to include a re-planning decision support system RDS. The re-planning decision support system RDS is a computerized module arranged in hardware, software or in both, that helps decide when for a given patient a re-planning may be necessary, specially, when such pre-planning is expected to yield a dosimetric benefit. The dosimetric benefit for an intended treatment re-planning can be defined as the difference between dose distributions as per the current plan and the re-planned plan. A dose distribution is a 3D scalar field that assigns to each spatial point in treatment space a local dosage value.

It may be appreciated that the treatment planning algorithm used at the planning module PM may be one of many. There exists a number of different re-planning techniques that can be used and the dose benefit may be contingent on which treatment technique one uses. However, common to most of these planning techniques is that they may draw considerable CPU time and hence energy, in particular when scaled up across large number of patients.

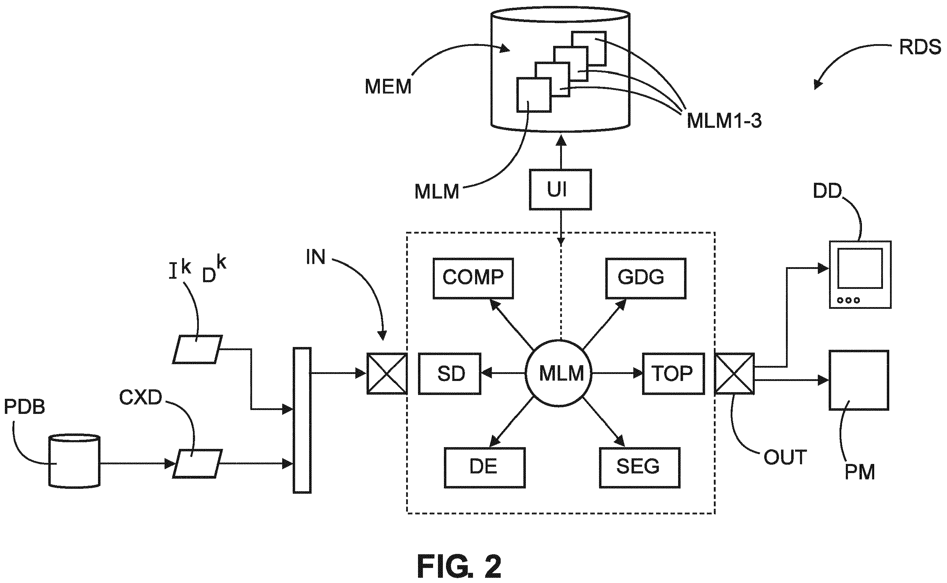

Reference is now made to the block diagram Fig. 2 to explain operation of computerized the radiation treatment re-planning support system RDS in more detail. The system RDS may be implemented as suitable software components on a computing unit PU, either general purpose or dedicated. Preferably, the decided or general purpose computing system PU may include one or more processors. In particular, one or more multi-core processors such as GPU (graphics processor unit) or TPU (tensor processing unit) may be used. A single one or a plurality of computing units may be used, possibly arranged as server(s) communicatively coupled in a communication network such as in a Cloud architecture. In particular, the computing for the machine learning component may be so outsourced to one or more servers. As an alternative to a purely software based implementation, some or all of the components of the system RDS may be implemented as hardware components such as FPGAs or ASICS.

Through one or more interfaces IN of the system RDS, a current fraction image $I^k$, or the initial planning image $I^0$, is received. A pre-trained machine learning module MLM then computes, at least based on this input image $I^k$, $I^0$, an estimated dose distribution that may be obtainable if, given the current situation as per the input, a re-planning is to be conducted based on a given planning technique.

This estimated dose distribution may be compared by a comparison module COMP with a dose distribution according to the current treatment plan to so quantify the expected dosimetric benefit. The dose distribution may be computed as a map that assigns to each image location a predicted dose deliverable. In other words, for 3D input imagery, the dose distribution is a 3D scalar field. One way to quantify the benefit is to form the point-wise difference to compute a difference map and compare this against a certain threshold. The comparison may be done globally for the whole of the two distributions, or may be done locally restricted to a certain sub-set(s) of interest. As will be explained more fully below, the machine learning module MLM to compute the dose distribution may be one of a plurality of such pre-trained machine leaning modules MLM 1-3 held in a memory. The user can then by user interface UI select which one of the machine learning modules should be used to predict the dose distribution to assess the dosimetric benefit. Preferably, each of the different pre-trained learning modules M1-3, MLM are associated with the respectively different planning techniques or algorithms. The user, can better assess which planning technique may bring about the (best) dosimetric benefit, if any. A user interface such as a graphical user interface allows the user to select from menus such as a drop down menu the respective planning technique. Upon selection an event handler intercepts the user requested command and provides the input received at the input interface IN to the selected one of the machine learning modules MLM 1-3 to compute the result $R$. The result $R$ is a quantification or indication of the estimated dose distribution. The result $R$ may be converted by the comparator COMP into a measure that quantifies the dosimetric benefit. The comparator COMP may be part of the machine learning module. The machine learning module may thus natively output the dosimetric benefit as result $R$. In addition or instead of testing different planning techniques in this manner, the machine modules may be associated with different treatment modalities such as photon therapy, electron therapy, or heavy particle therapy (e.g., carbon ion therapy, proton therapy). The user hence can assess which treatment modality may bring about a dosimetric benefit, if any.

The results computed by the machine learning module and/or the comparison result computed by comparator COMP may be graphically rendered by a graphics display generator GDG. For instance, the dose distribution may be color- or grey-value-coded and displayed on a display device DD. The estimated dose distribution may be displayed concurrently with the input imagery, such as side by side or superimposed.

Preferably and in addition, it is also the current dose distribution obtainable by the current plan that is concurrently displayed, side by side, superimposed or otherwise on the same display device DD or on a different one. The comparison result in relation to the two distributions, that is the dosimetric benefit, can be specifically highlighted by color- or grey value encoding, or may be otherwise indicated on in the graphics display such as by textual and/or numerical annotation boxes, superimposed on in the input image. The comparison result may be displayed as a single number globally for the whole distribution, or may be displayed individually per image element, such as per pixel or voxel element, or for a certain pre-defined sub-set region which the user demarcates by a suitable computer input tool such as computer mouse, stylus or otherwise. The user can then visually examine spatial aspects of the dosimetric benefit. The user may request other dosimetric benefits be computed based on other planning techniques or treatment modalities by selecting other machine learning modules MLM1-3.

The graphics display may be arranged as a graphical user interface (GUI) with interactive functionalities, such as planning image acquisition/ treatment re-planning bookings, or the said demarcation of sub-areas, new graphical renderings, the selection of machine learning modules/re-planning techniques etc.

The opposite of "dosimetric benefit" as understood herein is a "dosimetric loss" which represents the situation where there is in fact no improvement in dose delivery. A re-planning may thus in fact lead to worse dose distribution and that the current plan should be maintained. All reference herein to "dosimetric benefit" should hence be construed as "dosimetric benefit, if any". The user is thus provided by the proposed system RDS with an overview of the possible dosimetric benefits as per different planning techniques. The above described graphical rendering may be applied to each or selection of the results provided by the various machine learning modules MLM1-3 that the user chose to interrogate. Graphical renderings of the dosimetric benefits for different planning techniques may be displayed alongside, superimposed or otherwise on display device D by the graphics display generator GDG. The user can then thus ascertain the possible dose benefits to be had. If it is felt that an appreciable dosimetric benefit can be gained, the user can forward a request to the planning module PM by a scheduler SD to request a re-planning based on the respective planning technique that helps secure the highest dosimetric benefit. For present purposes a dosimetric benefit is established if the dose distribution as computed by any of the machine learning modules MLM, MLM1-3 indicates i) that the target would receive at least a dose as per the current plan and/or that the OAR receives a dose less than as per the current plan or ii) that the target would receive at higher dose as per the current plan and/or that the OAR receives a dose at most as per the current plan.

Specifically, the user can by mouse click or by other suitable interface means book a slot for re-planning and have the current information including the current fraction image forwarded to the planning module PM and placed into storage. Once a vacancy arises as per the schedule, the re-planning may commence. Instead, the re-planning may be requested automatically by the re-planning system RDS by examining the dosimetric benefit and if there is an appreciable positive dosimetric benefit as measureable against one or more thresholds, the re-planning request is issued automatically. The user can configure the system to customize the number and the value of dosimetric thresholds. The scheduler SD may be used to schedule a new imaging session to acquire a new planning image $I^{0'}$ at a suitable image planning modality IA1.

In embodiments, instead of merely predicting the dose distributions obtainable by re-planning, the re-planning decision system RDS may include a further machine learning module MLM' that allows predicting the anatomical changes given the current treatment plan and based on the current image $I^0$ or $I^j$ at a given fraction $j$. In other words, the anatomy trained machine learning module MLM' will predict a new image that is an estimate of the anatomy that may result at a selectable downstream fraction $F^i$, $i>j$.

As a further option, the re-planning decision system RDS may include a treatment output predictor TOP that estimates the treatment outcome for the patient given the current information at fraction $i$ or at planning stage $i=0$. The outcome may include side effects, tumor control and/or other. Whilst treatment outcome predictor TOP may itself be arranged as a pre-trained machine learning module, the predictor may instead by configured as a deterministic mapper such as a look-up table (LUT). In the LUT embodiment of the treatment outcome predictor TOP or similar deterministic mapper, a medical knowledge data base may be queried to match the current medical situation to outcome predictions as encoded in the medical knowledge data base.

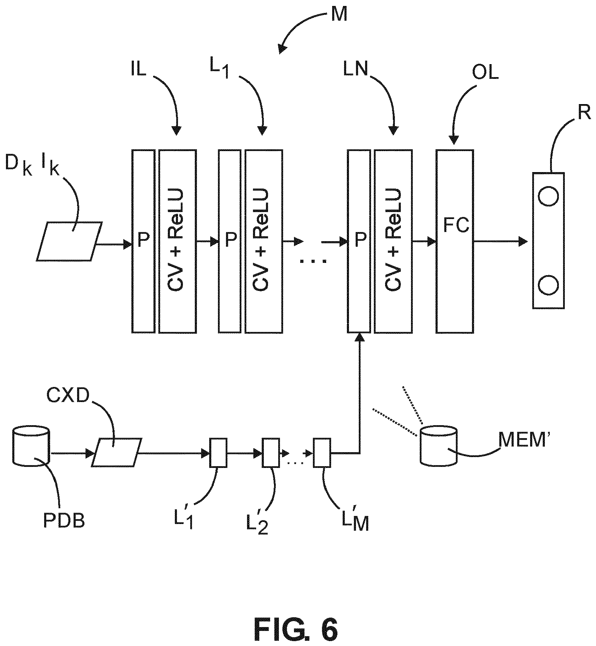

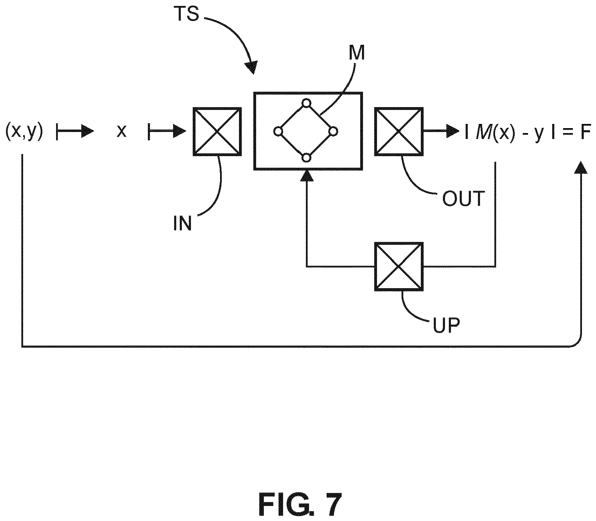

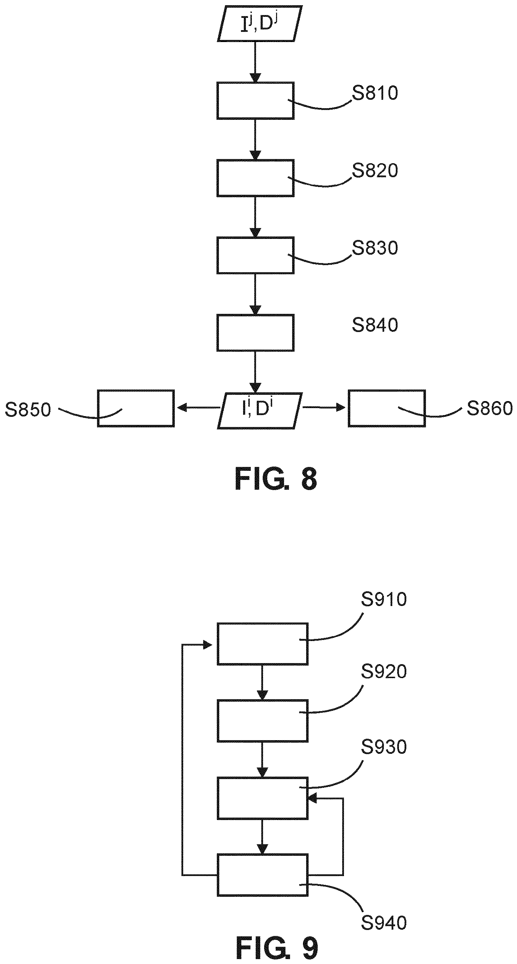

The various machine learning modules MLM, MLM1-3, MLM' and possibly TOP are all pre-trained on suitable training data in a training phase. After training phase, the machine learning models MLM, MLM1-3, MLM' and TOP are considered sufficiently trained and can then be used in deployment phase to be applied to new data not "seen" before, that is, new data not part of the training data. The training aspects will be described in more detail below with reference to Figs. 7 and 8, whilst Fig. 6 will provide more details on machine learning models M that may be used in the machine learning modules MLM, MLM1-3, MLM' and TOP as envisaged herein.

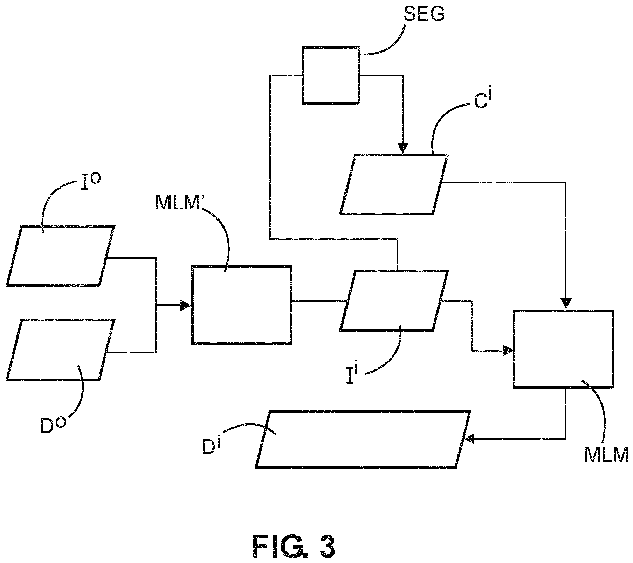

Turning now first to Figs. 3-5, other embodiments of the re-planning decision support system will now be described. In the following Figs. 3-5 it is assumed that the underlying machine learning models of the various machine learning modules have been sufficiently pre-trained on training data.

Referring now first to Fig. 3, this shows a schematic block diagram of the re-planning decision system RDS as may be used at the planning phase. The system allows the user, for example at the planning stage, to predict, via the pre-trained ML module MLM', a control image at a selectable fraction $i$ ($i$ = 1, ... ,$N$). The control image represents the patient anatomy at fraction $i$. Based on the predicted control imagery $I^i$, the corresponding re-planned) dose distribution can be predicted via another machine learning module MLM. The predicted dose distribution $D^i$ (referred herein also as "dose map") may result from re-planning, using a planning technique associated with the machine learning module MLM. The user can choose the appropriate model MLM, MLM1-3 by operation of the user interface UI as described above. The machine learning model has been preferably so trained that the predicted dose distribution is "optimal" in the sense that it comes close (within a margin) to a dose distribution obtainable in an actual re-planning.

The machine learning model MLM' that is used to predict the control image $I^i$ at fraction $i$ can be constructed using e.g., a Generative Adversarial Network (GAN) setup. GANs have been reported by Ian Goodfellow et al, "Generative Adversarial Networks", submitted on 10 June 2014, available online on the arXiv distribution service at https://arxiv.org/abs/1406.26.

As an alternative to neural network type models, previous work in radiation therapy demonstrates that statistical models trained on population patient data can also learn the patterns of anatomical changes during the course of fractionated radiation therapy. For a new patient, these models generate, at the planning phase, possible instances of the anatomy during the course of the treatment. Nevertheless, statistical models require descriptive models of anatomical changes. The advantage of using neural networks is that they do not require such a descriptive model.

The contours $C^i$ for the fraction $i$ anatomy can be generated by a segmentor algorithm SEG using deformable image registration. Alternatively, the segmentations themselves may be done by suitably trained ML models, such as NNs.

The system as described in Fig. 3 has a two-stage architecture with two ML models MLM, MLM' operating in series: one model MLM' predicts the control image, and the second model MLM predicts the re-plannable dose $D^i$ from the predicted control image $I^i$. However, such a cascaded serial architecture is not of necessity herein. A single ML model MLM may be used instead thus "leapfrogging" the control image $I^i$ prediction, to directly predict the re-plannable dose distribution $D^i$.

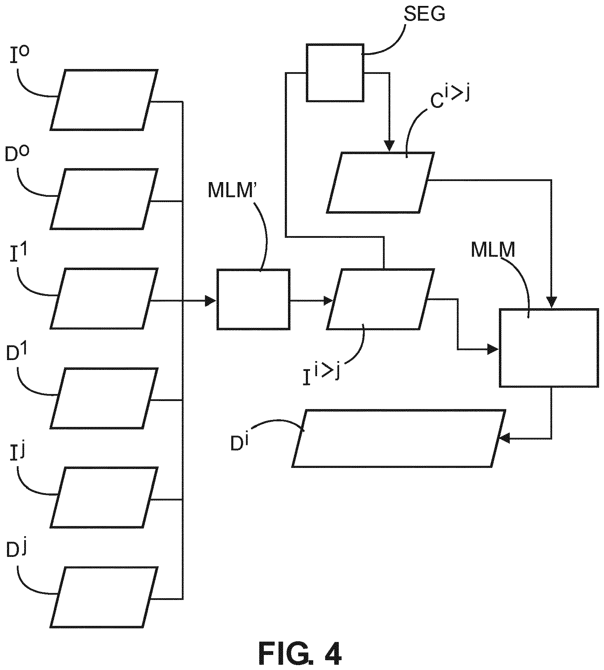

Reference is now made to the block diagram in Fig. 4 which shows the embodiment of the re-planning system RDS which may be used during treatment after planning phase. In more detail, the prediction of control image $I^i$ at fraction $i$ can be updated during the course of the treatment by using a machine learning module MLM. The module MLM takes as input any one or more (in particular all) of the planning image $I^0$, planning dose, earlier control imagery $I^j$, $j<i$, re-planned dose distributions $D^j$, $j<i$ of one or multiple fractions $j$ delivered prior to fraction $i$. The architecture is thus similar to the one in Fig. 3, but is used during treatment rather than (as in Fig. 3) at planning stage. Again, as mentioned in relation to Fig. 3, the leapfrogging architecture with a single ML module can be used to directly predict the dose $D^i$ due to re-planning, without computing first the anatomical control image $I^i$. Optionally, the planning of resources can be updated after day 1 by using the setup in Fig. 4.

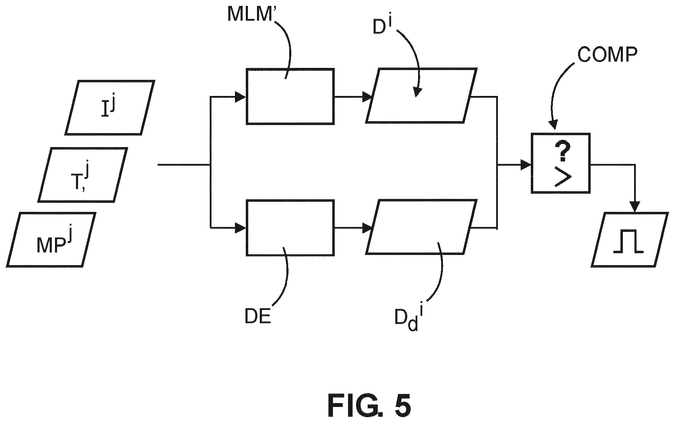

Reference is now made to Fig. 5 which explains in more detail the above described operation of the comparator COMP. The model MLM predicts for a given control image $I^j$ and a planning technique, the dose map $D^i$ for later fraction $i>$j. The dosimetric difference, a benefit or loss, between the fraction $i$ predicted optimal re-planned dose $D^i$ and the deliverable dose $D_d^i$ at fraction $i$ is computed by comparator COMP against on or more thresholds, in order to support decisions for treatment adaptation such as the acquisition of a new planning image $I^0$ and/or re-planning with the same or a different planning technique. A different planning technique with respect to the previous plan, can be for example, a plan with different beam angles, number of beams, or number of arcs (for VMAT)Based on this decision, a slot at the planning station PM or planning imager IA1 may be booked. The current deliverable dose map $D_d^i$ at fraction $i$ may be computed by a dose engine DE based on current control image $I^j$ current treatment plan $T^j$, and/or machine parameters $MP^j$. The dose engine implements dose calculation algorithms, such as e.g., pencil beam, collapsed cone, Monte-Carlo or other.

Operation of the optional of the scheduler SD may be illustrated as follows: suppose an RT department has $N$ patients planned to start treatment at a given day, say day "1". The user predicts the fraction $i$ images $I^i$ and the fraction $i$ dose distribution $D^i$ for the $N$ patients using the system RDS as describe above. For a number $q$ of patients out of the total of $N$ registered patients, the difference between the optimal predicted dose and the recalculated dose at fraction $i$ is larger than a clinically accepted threshold. Thanks to the pre-trained ML modules MLM', MLM, MLM1-3, the user can thus already plan $q$ image acquisition time slots to acquire new planning images $I^{0'}$, before next fraction $i$.

In embodiments, the proposed system provides to the user at the planning stage an overview of possible anatomical changes and dose deviations for a new patient. The user can prevent these dose degradations by robust plan optimization, can schedule at the treatment planning phase the time slot to acquire a CT-scan for re-planning, or can generate a library of plans encompassing possible anatomical changes. In embodiments, during the course of the treatment, the proposed system provides the user with quantitative information to support decisions on treatment adaptation. In robust plan optimization, uncertainties are factored into the optimization such the predicted anatomical changes. In other words, the proposed system provide the user with means to predict possible anatomical changes before the treatment and define uncertainties beforehand to ensure dose will be within the intended bounds. Robust planning has been described by T.C.Y Chan et al in "Adaptive and robust radiation therapy optimization for lung cancer", published in "European Journal of Operational Research", vol 231 (2013), pp 745-756.

Reference is now made to Fig. 6 which shows a schematic block diagram of a machine learning model M that may be used in any of the above described machine learning modules MLM, MLM1-3, MLM', TOP. Specifically, in embodiments, but not necessarily in all embodiments, a neural-network ("NN") type model may be used. In particular, and in embodiments, an at least partially convolutional neural-network type ("CNN") is used, which includes one or more layers that are non-fully connected layers. Fig 6 shows a schematic block diagram of a neural network M of the feed-forward type, but recurrent architectures are not excluded herein.

Conceptually, the operation of the machine learning model M as envisaged herein may be understood as follows. There may be a latent mapping $L$ or relationship between the dosage distributions as per a current treatment plan and control/target image $I^j, I^0$, and a dose distribution obtainable from a certain re-planning technique for a next fraction $i>j$, given current conditions, such as a current control image $I^i$. This mapping or relationship $L$ is latent in it is usually unknown and cannot be explicitly computed. The machine learning module during training by processing a plurality of training data allows one to adjust the parameters of the machine learning module to effectively learn, in approximation, this relationship $L$. The trained model M is an approximate representation of the latent mapping $L$ and allows one to make predictions on hypothetical dose distributions that may result from re-planning when provided with new imagery not part of the training imagery.

The model M may be trained by a computerized training systems TS to be described more fully below at Fig. 7. In training, the training system TS adapts an initial set of (model) parameters $\theta$ of the model M. In the context of neural network models, the parameters are sometime referred to herein as weights. The training data may be generated by simulation or may be procured from existing historic imagery or other data as may be found in medical image database such as in PACS (picture archiving and communication system) or similar as will be described in more detail below in relation to Fig. 7. Two processing phases may thus be defined in relation to the machine learning model NN: a training phase and a deployment (or inference) phase.

In training phase, prior to deployment phase, the model is trained by adapting its parameters based on the training data. Once trained, the model may be used in deployment phase to predict later dose distribution $D^j$ or later control imager $I^j$ due to re-planning based on a given planning technique, given current image $I^i$. The training may be a one-off operation, or may be repeated once new training data become available.

The machine learning model M may be stored in one (or more) computer memories MEM'. The pre-trained model M may be deployed as a machine learning component that may be run on a computing device PU, such as a desktop computer, a workstation, a laptop, etc or plural such devices in a distributed computing architecture. Preferably, to achieve good throughput, the computing device PU includes one or more processors (CPU) that support parallel computing, such as those of multi-core design. In one embodiment, GPU(s) (graphical processing units) are used.

Referring now in more detail to Fig. 6, this shows a convolutional neural network M in a feed-forward architecture. The network M comprises a plurality of computational nodes arranged in layers in a cascaded fashion, with data flow proceeding from left to right and thus from layer to layer. Recurrent networks are not excluded herein. Convolutional networks have been found to yield good result when processing image data.

In deployment, the input data $x$ is applied to input layer IL, such as $x=I_j$, $D_j$, optionally complemented with contextual non-image data CXD (on which more below). The input data $x$ then propagates through a sequence of hidden layers $L_1$-$L_N$ (only two are shown, but there may be merely one or more than two), to then emerge at an output layer OL as an estimate output $M(x)$. As per the embodiments of Figs. 2-5 described above, the output M(x) may be i) an indication on whether or not there is a dosimetric benefit for re-planning at follow-up/later fraction $i$, or ii) an indication of a clinical outcome, iii) later control image $I_i$ or iv) later dose map $D_i$. In case of iii),iv), the output data has the same size as the input date $x$.

The model network M may be said to have a deep architecture because it has more than one hidden layers. In a feed-forward network, the "depth" is the number of hidden layers between input layer IL and output layer OL, whilst in recurrent networks the depth is the number of hidden layers, times the number of passes.

The layers of the network, and indeed the input and output imagery, and the input and output between hidden layers (referred to herein as feature maps), can be represented as two or higher dimensional matrices ("tensors") for computational and memory allocation efficiency. The dimension and the number of entries represent the above mentioned size.

Preferably, the hidden layers include a sequence of convolutional layers, represented herein as layers $L_1$ - $L_N$. The number of convolutional layers is at least one, such as 2-5, or any other number. The number may run into double-digit figures.

In embodiments, downstream of the sequence of convolutional layers there may be one or more fully connected layers FC, in particular if a classification result is sought, such as in classification of whether or not there is a dosimetric benefit. However, if the input such as control image $I_j$ or dose map is to be regressed into later control image $I_i$ or a later dose map $Di$ as per a re-planning technique, no such fully connected layers may be required. In this case, the size of the input equals the size of the output.

Each hidden $L_m$ layer and the input layer IL implements one or more convolutional operators CV. Each layer $L_m$ may implement the same number of convolution operators CV or the number may differ for some or all layers.

A convolutional operator CV implements a convolutional operation to be performed on its respective input. The convolutional operator may be conceptualized as a convolutional kernel. It may be implemented as a matrix including entries that form filter elements (the said weights) that form at least a part of the model parameters $\theta$. It is in particular these weights that are adjusted in the learning phase. The first layer IL processes, by way of its one or more convolutional operators, the input data such as control image $I^j$, or dose map $D^j$. Feature maps are the outputs of convolutional layers, one feature map for each convolutional operator in a layer. The feature map of an earlier layer is then input into the next layer to produce feature maps of a higher generation, and so forth until the last layer OL represents the desired output $I^j$, or $D^i$. In classifications, last layer OL, an output feature map of one or more previous fully connected layers FC, represents the classification result, such as dosimetric benefit yes/no for later fraction $i$, clinical outcome result, etc, depending on the respective embodiment as described above in relation to Figs 2-5.

A convolutional layer $L_{1-N}$ is distinguished from a fully connected layer FC in that an entry in the output feature map of a convolutional layer is not a combination of all nodes received as input of that layer. In other words, the convolutional kernel is only applied to sub-sets of the input image/dose map $I^j$, $D^j$, or to the feature map as received from an earlier convolutional layer. The sub-sets are different for each entry in the output feature map. The operation of the convolution operator can thus be conceptualized as a "sliding" over the input, akin to a discrete filter kernel in a classical convolution operation known from classical signal processing. Hence the naming "convolutional layer". In a fully connected layer FC, an output node is in general obtained by processing all nodes of the input layer.

The stride of the convolutional operators can be chosen as one or greater than one. The stride defines how the sub-sets are chosen. A stride greater than one reduces the dimension of the feature map relative to the dimension of the input in that layer. A stride of one is preferred herein. In order to maintain the dimensioning of the feature maps to correspond to the dimension of the input imagery, a zero padding layer P may be applied in embodiments. This allows convolving even feature map entries situated at the edge of the processed feature map.

Whilst the machine learning module may operate purely on input imagery it may be helpful to provide further context in the form of the above mentioned contextual data CXD that is processed jointly with the image data. This contextual data CXD may include in particular non-image data such as bio-characteristics of the patient, for instance, age, sex, weight etc, ethnicity and/or indications of medical history, etc.

The contextual data CXD may be represented or encoded as vectors, matrices or tensors just like the imagery, however, processing such contextual data as if it were images by a convolutional system is unlikely to yield good results because correlations in non-image data may differ from the type of spatial correlations in neighboring pixels of image data for example.

When contextual data CXD is to be processed, it may be beneficial to use multi-strand processing where the non-contextual data are processed in a separate strand or processing path by one or more additional layers $L_1'$-$L_M'$, separate from the strand of layers $L_1$-$L_N$ used for the image data processing. The output of the final layer $L_M'$ in the strand of layers for contextual data CXD processing may be merged with the strand $L_1$-$L_N$ for processing of the image data. The layers in the contextual data processing strand $L_1'$-$L_M'$ are preferably one, two or more fully connected layers. Such fully connected layers have been found to be better suited to process non-image data. Non-image data, such as categorical data, may lead to sparseness in vector or matrix representation which may be undesirable. Embedding techniques may be used to encode the non-image data into a representation which can then be processed jointly with the image data. Such embeddings may include for example one-hot encoding schemes. In other embodiments, an auto-encoder setup is used in the non-image data processing models to obtain a suitable representation, a "code", which may be more suitable for co-processing with image data. The code is in general a lower dimensional representation with less sparsity that may be fed into the image processing strand as a "pseudo"-image where it can then be jointly processed with the image data, for example by concatenation. The strand for non-image contextual data is shown in the lower part of Fig. 6 as a separate processing strand $L_1'$-$L_M'$.

The main output result $R$ of the model M is provided at output layer may be image-like such as is the case when the hypothetical dose distribution due to re-planning is predicted. In his case, operation of the machine learning module MLM can be understood as one of regression where the input is regressed into the output image, such the 3D or 2D scalar field that is the dose distribution.

In alternative embodiments the model M is one of classification in particular when only a binary assessment is sought on whether or not there would be a dosimetric benefit on re-planning. In this embodiment, the output is classified into a vector with two entries indicating whether or not there is a dosimetric benefit. As a further variant instead of such a binary output, a probability distribution over two entries may be computed where the entries indicate whether or not there is a probability of a benefit. In an extreme case only a single number is provided at the output result, the output being the probability for whether a dosimetric benefit is obtainable. It is in particular in such classification setups that the model M may include, in addition to the one or more convolutional layers IL, $L_1$-$L_N$, one or more fully connected layers FC as mentioned above. In case of a classification result, the output layer OL may be configured as a *softmax*-function layer or as similar computational nodes where feature maps from previous layer(s) are combined into normalized counts to represent the classification probability per class.

It will be understood that the above described model M in Fig. 6 is merely according to one embodiment and is not limiting to the present disclosure. Other neural network architectures are also envisaged herein with more or less or different functionalities than describe herein, such as pooling layers or drop-out layers or others still. What is more, models M envisaged herein are not necessarily of the neural network type at all. Other, classical statistical regression methods based on sampling from training data are also envisaged herein in alternative embodiments. Still other techniques may include Bayesian networks, or random fields, such as Markov type random field and others.

The training system TS that may be used to train the machine learning model herein is now explained in more detail with reference to Fig 7.

In the above described architectures for CNN-type models M, the totality of the weights $W$ for all convolutional/deconvolutional filter kernels of the CNN model NN define a configuration of the machine learning model. The weights may differ for each layer and each layer may include plural convolutional operators some or each having a different set of kernels W. It is these weights $Wj$ that are learned in a training phase, where index $j$ runs over layers and convolutional operators therein. Once the training phase has concluded, the fully learned weights, together with the architecture in which the nodes are arranged, can be stored in one or more data memories MEM' and can be used for deployment.

In more detail, Fig. 7 which shows a training system TS for training the parameters, i.e. the weights of machine learning model such as in a convolutional neural network as discussed in Fig. 7 or other neural network-type model, or indeed non-neural network type ML models.

The training data comprises $k$ pairs of data $(x_k, y_k)$. k may run into the 100s or 1000s. The training data comprises for each pair $k$ (the index $k$ is not related to the index used above to designate generation of feature maps), training input data $x_k$ and an associated target $y_k$. The training data is thus organized in pairs $k$ in particular for supervised learning schemes as mainly envisaged herein. However, it should be noted that non-supervised learning schemes are not excluded herein.

The training input data $x_k$ may be obtained from historical image data acquired in the lab or previously in the clinic and held in image repositories, such as the PACS of a HIS (hospital information system) for instance. The targets $y_k$ or "ground truth" may represent for examples label, in particular of classification type models M. Such labels may be retrieved from PACS databases or other medical data repositories. Historic control imagery, one or more planning images, labels of regions of interest such as tumor and organs at risk, dose maps, number of fractions, dose per fraction, beam geometry (e.g., number of beams, angle of beams, shape of beams), etc are often stored in such repositories for historical RT treatments of earlier patients. The respective labels may be found in header data or could be inferred from inspecting the medical records and notes for the historic imagery and dose maps. The labels $y_k$ for training a classification type model may include any or more of: an indication of a clinical outcome, an indication for the dosimetric benefit for a given historic control image, dose map D stored for the given fraction i for the historic RT study. In such cases, pairs $(x_k, y_k)$ can be formed by querying data repositories, with $x_k$= a control image or dose map and $y_k$ the associated label for any one of clinical outcome, indication of dosimetric loss or benefit associated with a certain planning technique. Indications for the different planning techniques used in the historical studies may also be found in the medical records of radiotherapy treatment data.

In regression type models M, such as when the follow up/later dose map $D^i$ or control image $I^k$ are to be predicted, the training data pairs $(x_k, y_k)$ are formed as $x_k$ = a control image or dose map for planning image/dose map $j^i$, $D^j$ for earlier fraction $j$ and the respective targets being later $i$ control imagery/dose maps $i^i$, $D^i$ or re-planned images $I^{0'}$, doses $D^{i0'}$. The training data may need to be grouped according to the respective planning techniques used to for training the respective models MLM, MLM1-3.

It is not necessary that all imagery/dose maps pertain to the same patient. Different pairs k may relate to different patients in general.

If training is to include contextual data CXD, there is in general no contextual data included in the target $y_k$ for any pair $k$, such as in the multi-strand models as discussed above in relation to Fig 6. In other words, for learning with contextual data the pairs may in general have a form $((x_k,c),y_k)$, with non-image context data $c$ only associated with the training input $x_k$, but not with the target $y_k$.

In the training phase, an architecture of a machine learning model M, such as the shown CNN network in Fig 6 is pre-populated with initial set of weights. The weights $\theta$ of the model NN represent a parameterization $M^\theta$, and it is the object of the training system TS to optimize and hence adapt the parameters $\theta$ based on the training data $(x_k, y_k)$ pairs. In other words, the learning can be formulized mathematically as an optimization scheme where a cost function F is minimized although the dual formulation of maximizing a utility function may be used instead.

Assuming for now the paradigm of a cost function F, this measures the aggregated residue(s), that is, the error incurred between data estimated by the neural network model NN and the targets as per some or all of the training data pairs $k$:

$$argmin_{\theta} F = \sum_k \| M^\theta(x_k) - y_k \| \qquad (1)$$

In eq. (1) and below, function $M()$ denotes the result of the model NN applied to input $x$. The cost function may be pixel/voxel-based, such as the L1 or L2-norm cost function. The Euclidean-type cost function in (1) (such as least squares or similar) may be used for the above mentioned regression task when computing anatomical changes or dose distributions. When configuring the model as a classifier to predict dosimetry gain/benefit, the summation in (1) is formulated instead as one of cross-entropy or Kullback-Leiber divergence or similar.

In training, the training input data $x_k$ of a training pair is propagated through the initialized network M. Specifically, the training input $x_k$ for a $k$-th pair is received at an input IL, passed through the model and is then output at output OL as output training data $M^\theta(x)$. A suitable measure $\|\cdot\|$ is used such as a $p$-norm, squared differences, or other, to measure the difference, also referred to herein as residue, between the actual training output $M^\theta(x_k)$ produced by the model M, and the desired target $y_k$.

The output training data $M(x_k)$ is an estimate for target $y_k$ associated with the applied input training image data $x_k$. In general, there is an error between this output $M(x_k)$ and the associated target $y_k$ of the presently considered $k$-th pair. An optimization scheme such as backward/forward propagation or other gradient based methods may then be used to adapt the parameters $\theta$ of the model M so as to decrease the residue for the considered pair $(x_k, y_k)$ or a subset of training pairs from the full training data set.

After one or more iterations in a first, inner, loop in which the parameters $\theta$ of the model are updated by updater UP for the current pair $(x_k, y_k)$, the training system TS enters a second, an outer, loop where a next training data pair $x^{k+1}, y^{k+1}$ is processed accordingly. The structure of updater UP depends on the optimization scheme used. For example, the inner loop as administered by updater UP may be implemented by one or more forward and backward passes in a forward/backpropagation algorithm. While adapting the parameters, the aggregated, for example summed, residues of all the training pairs are considered up to the current pair, to improve the objective function. The aggregated residue can be formed by configuring the objective function F as a sum of squared residues such as in eq. (1) of some or all considered residues for each pair. Other algebraic combinations instead of sums of squares are also envisaged.

Optionally, one or more batch normalization operators ("BN", not shown) may be used. The batch normalization operators may be integrated into the model M, for example coupled to one or more of the convolutional operator CV in a layer. BN operators allow mitigating vanishing gradient effects, the gradual reduction of gradient magnitude in the repeated forward and backward passes experienced during gradient-based learning algorithms in the learning phase of the model M The batch normalization operators BN may be used in training, but may also be used in deployment.

The training system as shown in Fig. 7 can be considered for all learning schemes, in particular supervised schemes. Unsupervised learning schemes may also be envisaged herein in alternative embodiments. GPUs may be used to implement the training system TS.

The fully trained machine learning module M may be stored in one or more memories MEM' or databases, and can be made available as pre-trained machine learning modules MLM, MLM1-3, MLM', TOP. The trained modules may be made available in a cloud service. Access can either be offered free of charge or their use can be granted via license-pay or pay-per-use scheme.

Reference is now made to the flow chart in Fig. 8 which shows a method for supporting radiation therapy.

At step S810 an input image is received. The input image may be control image $I^k$ at fraction $k$, or may be a planning image $I^0$. Optionally, in addition, contextual data, such as bio-characteristics of the patient to whom the image pertains, is received.

At step S820 a pre-trained machine learning module is applied to the input image to produce a predicted dose distribution. The input image relates to the current treatment plan, in particular a current planning technique that was used to compute the current treatment plan. The predicted dose distribution is a 3D or 2D scalar field, and is associated with a certain planning technique. The predicted dose distribution represents the dose distributions that may be obtained if the said planning technique would be used to re-plan for a new treatment plan. The associated planning technique may be the same as the current planning technique/algorithm, or may be a different planning technique. The control image $I^k$ may be 2D as it may have been acquired with a low dose radiography modality such as the earlier mentioned in-room kV-imager IA2, in which case the predicted dose map is preferably likewise 2D. In case the initial planning image $I^0$ is used, this is preferably 3D and then so is the predicted dose.

At step S830 the predicted dose distribution is compared with the current dose distribution as per the current treatment plan to obtain a comparison result. The result may be an absolute difference point-wise between the two distributions or may be otherwise processed into a weighted difference or the two distributions are otherwise combined to produce the result R. The result R might be a weighted difference of a dose statistic (e.g., min max, dose volume histogram, etc.) corresponding to one or more regions of interest.

At step S840 the predicted dose distributions and/or the comparison result is displayed in a display device, is stored, transmitted, or otherwise processed.

At optional step S850, in response to a user request or, based on the comparison result, a re-planning is requested for a new treatment plan. The same or a different planning technique may be used. In addition or instead, a new treatment modality may be used and the planning is based on this new treatment modality.

At step S860, a re-planning at a planning station is scheduled. In addition or instead, acquisition of a new planning image $I^{0'}$ is requested. Optionally, the new treatment modality is booked.

Optionally, there is a selection step S820 where the machine learning model used in step S850 is selected from a plurality of pre-training machine learning modules. Each of the pre-trained modules held in storage is associated with different a planning technique. The selection at step S820 is either made automatically or is based on a user selection. A plurality of different such selections can be made. The user can hence compute multiple expected estimated dose distributions, each associated with different planning algorithms to so have a better overview of all possible dosimetric benefits potentially achievable. Each or a sub-selection of the multiple of the expected estimated dose distributions can be displayed, concurrently or in sequence, optionally with the respective dosimetric benefit graphically, textually or numerically indicated in the graphics display as generated at step S840. As mentioned, the re-planing with the same of different technique may be for a different treatment modality than the one currently used. The pre-trained modules may thus be associated not only with different planning technique but also with different treatment modalities so the user can explore dosimetric gains with respect to different treatment modalities. Alternatively, instead of being associated with planning techniques, the pre-trained modules are associated with the different treatment modalities.

In a further option, at step S840, in addition to the dose distribution, an image $I^j$ that represents the anatomy at the i-th fraction given the current treatment plan is predicted.

In a further option, a treatment outcome is predicted given the current treatment plan. The dose distribution, and the optional anatomy and/or clinical outcome may be computed by the same machine learning model ("ML") model, or by respective different models, suitably trained on respective data, including image data and optionally non-image contextual data.

Reference is now made to Fig. 9, which shows a flow chart of a method of training a machine learning model for computerized radiation therapy support.

Suitable training data needs to be collated. Preferably, supervised learning schemes are envisaged herein although this is not a necessity as unsupervised learning setups are also envisaged herein.

In supervised learning, the training data includes suitable pairs of data items, each pair including training input data and associated therewith a target training output data. Specifically, the pairs comprise. The imagery or the projection data can be paired up by retrieving the same from historic RT treatment data records such as a PACS or other data repository, as described above.

With continued reference to Fig. 9, at step S910 training data is received in the form of pairs $(x_k, y_k)$. Each pair includes the training input $x_k$ and the associated target $y_k$. $x_k$, as defined in Fig. 7 above.

At step S920, the training input $x_k$ is applied to an initialized machine learning model NN to produce a training output.

A deviation, or residue, of the training output $M(x_k)$ from the associated target $y_k$ is quantified by a cost function $F$. One or more parameters of the model are adapted at step S930 in one or more iterations in an inner loop to improve the cost function. For instance, the model parameters are adapted to decrease residues as measured by the cost function. The parameters include in particular weights W of the convolutional operators, in case a convolutional NN model M is used.

The training method then returns in an outer loop to step S910 where the next pair of training data is fed in. In step S920, the parameters of the model are adapted so that the aggregated residues of all pairs considered are decreased, in particular minimized. The cost function quantifies the aggregated residues. Forward- backward propagation or similar gradient-based techniques may be used in the inner loop.

More generally, the parameters of the model NN are adjusted to improve objective function $F$ which is either a cost function or a utility function. In embodiments, the cost function is configured to the measure the aggregated residues. In embodiments the aggregation of residues is implemented by summation over all or some residues for all pairs considered. The method may be implemented on one or more general-purpose processing units TS, preferably having processors capable for parallel processing to speed up the training.

The components of the training system TS may be implemented as one or more software modules, run on one or more general-purpose processing units PU such as a workstation associated with the imager IA1, IA2, or on a server computer associated with a group of imagers.

Alternatively, some or all components of the training system TS may be arranged in software or in hardware. The hardware may include a suitably programmed microcontroller or microprocessor, such an FPGA (field-programmable-gate-array) or as a hardwired IC chip, an application specific integrated circuitry (ASIC), integrated into the imaging system NIR. In a further embodiment still, the training system TS may be implemented in both, partly in software and partly in hardware.

The training system TS may be implemented on a single data processing unit PU. Alternatively, some or more components are implemented on different processing units PU, possibly remotely arranged in a distributed architecture and connectable in a suitable communication network such as in a cloud setting or client-server setup, etc.

One or more features described herein can be configured or implemented as or with circuitry encoded within a computer-readable medium, and/or combinations thereof. Circuitry may include discrete and/or integrated circuitry, a system-on-a-chip (SOC), and combinations thereof, a machine, a computer system, a processor and memory, a computer program.

In another exemplary embodiment of the present invention, a computer program or a computer program element is provided that is characterized by being adapted to execute the method steps of the method according to one of the preceding embodiments, on an appropriate system.

The computer program element might therefore be stored on a computer unit, which might also be part of an embodiment of the present invention. This computing unit may be adapted to perform or induce a performing of the steps of the method described above. Moreover, it may be adapted to operate the components of the above-described apparatus. The computing unit can be adapted to operate automatically and/or to execute the orders of a user. A computer program may be loaded into a working memory of a data processor. The data processor may thus be equipped to carry out the method of the invention.

This exemplary embodiment of the invention covers both, a computer program that right from the beginning uses the invention and a computer program that by means of an up-date turns an existing program into a program that uses the invention.

Further on, the computer program element might be able to provide all necessary steps to fulfill the procedure of an exemplary embodiment of the method as described above.

According to a further exemplary embodiment of the present invention, a computer readable medium, such as a CD-ROM, is presented wherein the computer readable medium has a computer program element stored on it which computer program element is described by the preceding section.

A computer program may be stored and/or distributed on a suitable medium (in particular, but not necessarily, a non-transitory medium), such as an optical storage medium or a solid-state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further exemplary embodiment of the present invention, a medium for making a computer program element available for downloading is provided, which computer program element is arranged to perform a method according to one of the previously described embodiments of the invention.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

**Claims**

1. A computing system for supporting radiation therapy, comprising:

   - an input interface (IN) for receiving an input image;
   - a machine learning module (MLM) to predict based on the input image, a predicted dose distribution associated with a first planning technique or first treatment modality; and
   - a comparator (COMP) configured to compare a planned dose distribution as per a current treatment plan with the predicted dose distribution, to obtain a comparison result.

2. System of claim 1, including a graphics display generator (GDG) configured to cause a display device (DD) to display the comparison result or data derivable therefrom.

3. System of claim 1 or 2, wherein the said comparison result is displayed in association with the input image.

4. System of claim 3, wherein the said comparison result is displayed globally for the whole input image or locally per image element.

5. System of any one of the previous claims, wherein, in response to the comparison result, or in response to a user request, a re-planning module (PM) to compute a new treatment plan.

6. System of any one of the previous claims, including a scheduler (SD) configured to schedule a new image session and/or a new re-planning session using the same or a planning technique, and/or new treatment session with the same or a new treatment modality.

7. System of any one of the previous claims, wherein the machine learning module (MLM) is one of a plurality of such modules (MLM,MLM1-3), with different ones of the said plurality of machine learning modules respectively associated with different planning techniques and/or different treatment modalities, the said modules held in one or more data memories (MEM).

8. System of claim 7, comprising a user interface (UI) for the user to select a different machine learning module (MLM) from the said plurality, and the system to produce a new comparison result, based on the selected machine learning module (MLM).

9. System of any one of the previous claims, the machine learning module (MLM), or a further machine learning module (MLM'), configured to predict an image representing anatomical changes due to applicable radiation fractions.

10. System of any one of the previous claims, wherein the comparison result is used by a treatment outcome predictor (TOP) to estimate a treatment outcome.

11. A computing system for training, based on training data, a machine learning module as per any one of the previous claims.

12. A computer-implemented method for supporting radiation therapy, comprising:

   - receiving (S810) an input image;
   - with a machine learning module (MLM), predicting (S820), based on the input image, a predicted dose distribution associated with a first planning technique and/or first treatment modality; and

- comparing (S830) a planned dose distribution as per a current treatment plan with the predicted dose distribution, to obtain a comparison result.

13. A computer-implemented method of training, based on training data, a machine learning module as per any one of previous claims.

14. A computer program element, which, when being executed by at least one processing unit, is adapted to cause the processing unit to perform the method as per any one of claims 11 and 12.

15. At least one computer readable medium (MEM') having stored thereon the program element of claim 13, or having stored thereon the pre-trained machine learning module as per in any one of the previous claims.

**FIG. 1**

**FIG. 2**

**FIG. 3**

$I^0$

$D^0$

$I^1$

$D^1$

$I^j$

$D^j$

SEG

$C^{i>j}$

MLM'

MLM

$I^{i>j}$

$D^i$

# FIG. 4

MLM'

$D^i$

COMP

$I^j$

$T_{,}^j$

$MP^j$

?
>

DE

$D_d{}^i$

# FIG. 5

**FIG. 6**

**FIG. 7**

FIG. 8

FIG. 9

Europäisches
Patentamt

European
Patent Office

Office européen
des brevets

# EUROPEAN SEARCH REPORT

Application Number

EP 20 18 4936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/095043 A1 (CORDERO MARCOS MARÍA ISABEL [FI] ET AL) 2 April 2015 (2015-04-02) | 1,2,7,8, 11-15 | INV. A61N5/10 |
| Y | * abstract * * paragraph [0025] - paragraph [0027] * * paragraph [0036] - paragraph [0114] * ----- | 3,4,9,10 | |
| X | EP 3 628 372 A1 (VARIAN MEDICAL SYSTEMS INT AG [CH]) 1 April 2020 (2020-04-01) * abstract * * paragraph [0050] - paragraph [0088] * ----- | 11,13-15 | |
| X | WO 2019/027924 A1 (ICAHN SCHOOL MED MOUNT SINAI [US]) 7 February 2019 (2019-02-07) | 11,13-15 | |
| Y | * abstract * * paragraph [0069] - paragraph [0089] * ----- | 3,4 | |
| X | WO 2018/048575 A1 (ELEKTA INC [US]) 15 March 2018 (2018-03-15) * the whole document * ----- | 11,13-15 | |
| X | EP 3 520 859 A1 (VARIAN MEDICAL SYSTEMS INT AG [CH]) 7 August 2019 (2019-08-07) * the whole document * ----- | 1,5,6,12 | TECHNICAL FIELDS SEARCHED (IPC)<br><br>A61N |
| Y | EP 3 576 100 A1 (SIEMENS HEALTHCARE GMBH [DE]) 4 December 2019 (2019-12-04) * abstract * * paragraph [0160] - paragraph [0173] * ----- | 9 | |
| Y | EP 3 673 955 A1 (KONINKLIJKE PHILIPS NV [NL]) 1 July 2020 (2020-07-01) * abstract * * paragraph [0113] * ----- | 10 | |

-/--

The present search report has been drawn up for all claims

| Place of search | Date of completion of the search | Examiner |
|---|---|---|
| Munich | 4 December 2020 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 20 18 4936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2015/176011 A1 (UNIV JOHNS HOPKINS [US]) 19 November 2015 (2015-11-19) * abstract * * paragraph [0210] - paragraph [0274] * | 10 | |
| A | MARY FENG ET AL: "Machine Learning in Radiation Oncology: Opportunities, Requirements, and Needs", FRONTIERS IN ONCOLOGY, vol. 8, 17 April 2018 (2018-04-17), XP055732929, DOI: 10.3389/fonc.2018.00110 * the whole document * | 1-15 | |

TECHNICAL FIELDS
SEARCHED          (IPC)

The present search report has been drawn up for all claims

| Place of search | Date of completion of the search | Examiner |
|---|---|---|
| Munich | 4 December 2020 | Beck, Ewa |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

& : member of the same patent family, corresponding
     document

EPO FORM 1503 03.82 (P04C01)

## ANNEX TO THE EUROPEAN SEARCH REPORT
## ON EUROPEAN PATENT APPLICATION NO.

EP 20 18 4936

This annex lists the patent family members relating to the patent documents cited in the above-mentioned European search report.
The members are as contained in the European Patent Office EDP file on
The European Patent Office is in no way liable for these particulars which are merely given for the purpose of information.

04-12-2020

| Patent document cited in search report | | Publication date | Patent family member(s) | | Publication date |
|---|---|---|---|---|---|
| US 2015095043 | A1 | 02-04-2015 | NONE | | |
| EP 3628372 | A1 | 01-04-2020 | CN | 110960803 A | 07-04-2020 |
| | | | EP | 3628372 A1 | 01-04-2020 |
| WO 2019027924 | A1 | 07-02-2019 | EP | 3661597 A1 | 10-06-2020 |
| | | | US | 2020155868 A1 | 21-05-2020 |
| | | | WO | 2019027924 A1 | 07-02-2019 |
| WO 2018048575 | A1 | 15-03-2018 | AU | 2017324627 A1 | 02-05-2019 |
| | | | CN | 109843377 A | 04-06-2019 |
| | | | EP | 3509697 A1 | 17-07-2019 |
| | | | JP | 6688536 B2 | 28-04-2020 |
| | | | JP | 2019526380 A | 19-09-2019 |
| | | | US | 2019192880 A1 | 27-06-2019 |
| | | | WO | 2018048575 A1 | 15-03-2018 |
| EP 3520859 | A1 | 07-08-2019 | CN | 110101974 A | 09-08-2019 |
| | | | EP | 3520859 A1 | 07-08-2019 |
| | | | US | 2019232087 A1 | 01-08-2019 |
| EP 3576100 | A1 | 04-12-2019 | CN | 110556178 A | 10-12-2019 |
| | | | EP | 3576100 A1 | 04-12-2019 |
| | | | US | 2019371450 A1 | 05-12-2019 |
| EP 3673955 | A1 | 01-07-2020 | EP | 3673955 A1 | 01-07-2020 |
| | | | WO | 2020136028 A1 | 02-07-2020 |
| WO 2015176011 | A1 | 19-11-2015 | US | 2017083682 A1 | 23-03-2017 |
| | | | WO | 2015176011 A1 | 19-11-2015 |

EPO FORM P0459

For more details about this annex : see Official Journal of the European Patent Office, No. 12/82

# EP 3 936 187 A1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. M. MITCHELL.** Machine Learning. McGraw-Hill, 1997
- **PFLUGFELDER et al.** A comparison of three optimization algorithms for intensity modulated radiation therapy. *Z. Med. Phys.,* 2008, vol. 18 (2), 111-119
- **IAN GOODFELLOW et al.** available online on the arXiv distribution service at. *Generative Adversarial Networks,* 10 June 2014
- **T.C.Y CHAN et al.** Adaptive and robust radiation therapy optimization for lung cancer. *European Journal of Operational Research,* 2013, vol. 231, 745-756